# EUROPEAN PATENT SPECIFICATION

(11) **EP 4 424 335 B1**
(45) Date of publication and mention of the grant of the patent: **30.09.2026**
(21) Application number: 22886817.0
(22) Date of filing: 19.10.2022
(51) Int. Cl.: A61L 101/22, A61L 2/22, A61L 9/14, F24F 7/06, B01L 1/00, B25J 21/02

(54) **DECONTAMINATION SYSTEM**
DEKONTAMINATIONSSYSTEM
SYSTÈME DE DÉCONTAMINATION

(30) Priority: 25.10.2021 JP 2021173858
(43) Date of publication of application: 04.09.2024
(73) Proprietor: Airex Co., Ltd., Nagoya-shi Aichi 453-0015 (JP)
(72) Inventor: KAWASAKI, Koji, Nagoya-shi Aichi 453-0015 (JP); KAKUDA, Daisuke, Nagoya-shi Aichi 453-0015 (JP); OGATA, Yoshitaka, Nagoya-shi Aichi 453-0015 (JP); KITAHORA, Kazuhiko, Nagoya-shi Aichi 453-0015 (JP); FUTAMURA, Haruka, Nagoya-shi Aichi 453-0015 (JP); GUO, Zhiqiang, Nagoya-shi Aichi 453-0015 (JP); KITANO, Tsukasa, Nagoya-shi Aichi 453-0015 (JP)
(74) Representative: Kraus & Lederer PartGmbB
(86) International application number: PCT/JP2022/038883
(87) International publication number: WO 2023/074489

(56) References cited:
- WO-A1-2021/090661
- CA-A1- 3 159 755
- JP-A- 2008 113 727
- JP-A- 2019 000 028
- JP-A- 2019 145 228
- JP-B2- 4 734 216
- JP-B2- 6 738 303

## Description

### TECHNICAL FIELD

The present invention relates to a decontamination system for decontaminating an air supply space constructed between an air filter for converting air supplied to an inside of a work room which maintains a sterile environment into clean air, and a straightening plate for straightening the clean air and supplying the straightened clean air to the inside of the work room.

### BACKGROUND ART

In works performed in a clean atmosphere, e.g., a work at a manufacturing stage of pharmaceutical products, or a work at a manufacturing stage of semiconductors or electronic components, the work is performed in a clean working environment which maintained an inside thereof in sterile isolator and dust-free conditions so that no contaminant enters from an external environment. As such a working environment, a clean room is generally used. In this clean room, an operator wearing dust-free clothes works. However, in order to increase a sterility assurance level and a dust-free assurance level, a still higher-level clean area is constructed in the clean room to perform the works.

One method of constructing such a higher-level clean area is to use an isolator or glove box (hereinafter collectively referred to as an "isolator"). This isolator uses a chamber sealed from the external environment, the operator works via a work glove or the like from outside this chamber. In the chamber of these isolators, a unidirectional laminar flow of clean air is flowed from an upper side to a lower side to maintain a clean environment in the chamber.

Moreover, a Restricted Access Barrier System (RABS) is used as another method of constructing a higher-level clean area. This RABS is configured to provide a area surrounded by a wall surface of which a lower portion is opened at a portion in the clean room, a unidirectional laminar flow of clean air is flowed into the inside thereof from an upper side to a lower side, and operator's strict access restrictions are realized. In this RABS, the operator works via a work glove or the like provided in the wall surface.

Moreover, in GMP (Good Manufacturing Practice; Good Manufacturing Practice Guidance for Active Pharmaceutical Ingredients), which summarizes requirements for manufacturing pharmaceutical products, a requirement for wind speed distribution with respect to clean air flow in the isolator and RABS has been stricter.

In the chamber of the isolator or the work room of RABS, a method of flowing the clean air from an upper side to a lower side is generally to install a punching plate on a ceiling portion in the chamber, through which the clean air that has passed through an air filter such as a HEPA filter is supplied from the upper side and exhausted downward. However, the opening of this punching plate has a pore size of several millimeters, and the Reynolds number of the airflow passing through is on the order of the fourth power of 10. In this state, the airflow becomes a turbulent flow from a stage of passing through the fine pores of the punching plate, and therefore a unidirectional steady flow flowing from the upper side to the lower side cannot be formed.

Therefore, instead of the punching plate, a straightening plate covered with a screen mesh having an opening of approximately 30 to 200 µm is used. In accordance with this method, the Reynolds number of the airflow passing through the screen mesh is approximately 2 to 10, and a unidirectional steady flow that is substantially a laminar flow can be formed.

On the other hand, hydrogen peroxide gas has been widely adopted into decontamination in the chamber of the isolator or the work room of RABS (hereinafter referred to as a "room targeted to be decontaminated"). Advantageously, hydrogen peroxide gas has a strong sterilization effect, and is inexpensively available and effectively utilized as an environmentally-friendly decontamination gas that is ultimately decomposed into oxygen and water. In addition, the following patent document 1 describes that the decontamination effect by hydrogen peroxide is provided by a condensed film of a hydrogen peroxide solution that condenses on the surface of an object to be decontaminated.

The inventors of the present invention have found that a fine mist of hydrogen peroxide solution (hereinafter referred to as a "hydrogen peroxide mist"), instead of a hydrogen peroxide gas, is supplied to a room targeted to be decontaminated, thereby achieving efficient decontamination using a least possible amount of decontamination agent, and have proposed the technique as described in Patent Literature 2 listed below. After that, decontamination using hydrogen peroxide mist has spread worldwide.

When decontaminating a room targeted to be decontaminated using hydrogen peroxide mist, the hydrogen peroxide mist is basically supplied to the room targeted to be decontaminated. At this time, there has been a problem that a space between an air filter and a straightening plate above the room targeted to be decontaminated (hereinafter referred to as "air supply space") is not sufficiently decontaminated, since it is principle that air is not circulated during the decontamination. In other words, the hydrogen peroxide mist discharged to the room targeted to be decontaminated cannot pass through the screen mesh and therefore cannot reach the air supply space. Moreover, there has been a problem that even if the hydrogen peroxide mist is directly supplied to the air supply space, dew condensation occurs on a surface of the screen mesh.

In order to solve this problem, it is considered to construct a circulation path of a hydrogen peroxide mist, from a hydrogen peroxide mist generating device to the room targeted to be decontaminated through the air supply space, and returning from the room targeted to be decontaminated to the hydrogen peroxide mist generating device. However, in this method, there has been a problem that a capacity of the circulation path is large, the amount of consumption of the hydrogen peroxide is increased, and decontamination cycle time is significantly extended.

Moreover, the inventors have proposed to additionally provide a hydrogen peroxide mist generating device and a mist dispersion/diffusion device in the air supply space in Patent Literature 3 listed below. Herein, the mist dispersion/diffusion device is configured with an ultrasonic vibration plate and to disperse and diffuse the hydrogen peroxide mist by acting acoustic radiation pressure due to an ultrasonic vibration on the hydrogen peroxide mist. However, in this method, there is also a problem that a hydrogen peroxide mist generating device must be installed in both the room targeted to be decontaminated and the air supply space, and the mist dispersion/diffusion device must also be installed in the air supply space, and therefore a mechanism thereof is complexed. Furthermore, in a small-scaled isolator, the air supply space may be small, and therefore it may be difficult to install the hydrogen peroxide mist generating device and the like therein.

CA 3159755 A1 discloses a decontamination system which does not require large-scale equipment such as a large-diameter duct and a condensation preventing heater, makes long-distance piping possible for each of a plurality of rooms to be decontaminated. JP 4734216 B2 discloses a decontamination system that supplies a decontamination gas such as a hydrogen peroxide gas into a decontamination room (for example, a clean room, an isolator, a chamber, or the like) to decontaminate the decontamination room. JP 6738303 B2 dicloses an isolator that handles pathogens and the like in an isolated space.

### CITATION LIST

### PATENT LITERATURE

Patent Document 1: JP-B-S61-4543
Patent Document 2: JP-A-2020-156970
Patent Document 3: WO-A1-2021-090661

### SUMMARY OF INVENTION

### TECHNICAL PROBLEM

Accordingly, the present invention has been made in order to solve the problems described above, and an object of the present invention is to provide a decontamination system having a simple structure using a decontamination agent supplied to a room targeted to be decontaminated without additionally providing a decontamination agent discharging device or the like in a space (air supply space) between an air filter and a straightening plate, the decontamination system being capable of efficiently decontaminating the air supply space in a short period of time.

### SOLUTION TO PROBLEM

The present invention relates to the claims 1 and 2. To solve the aforementioned problem, inventors of the present invention have carried out an extended investigation to find the advantage of using a decontamination agent transfer device configured to suction, discharge, or circulate decontamination mist between the room targeted to be decontaminated and the air supply space. Based on that technique, the present invention has been accomplished.

Specifically, an isolator device according to the present invention is, according to description in claim 1,
an isolator device comprising a decontamination system for decontaminating an air supply space constructed between an air filter for converting air supplied to an inside of a work room which is configured to maintain a sterile environment into clean air, and a straightening plate for straightening the clean air and supplying the straightened clean air to the inside of the work room,
further comprising said work room, said air supply space, said air filter, and said straightening plate;
the decontamination system comprising a decontamination agent discharging device provided in the work room, and a decontamination agent transfer device provided in the air supply space, characterized in that
the decontamination system is configured so that when decontaminating the inside of the work room, the decontamination agent discharging device is operated to discharge a decontamination agent to the inside of the work room, and the decontamination agent transfer device is operated to suction, discharge, or circulate the decontamination agent between the work room and the air supply space,
and thereby the air supply space is decontaminated as well as the work room;
characterized in that
the straightening plate comprises one or more porous sheets for straightening the clean air, and
the decontamination agent transfer device comprises an air duct having one end portion as a suction port and the other end portion as a discharge port, and an air-blowing fan provided inside the air duct, and is disposed so that the suction port is in contact with the porous sheet at an inside side of the air supply space.

Furthermore, the present invention is, according to description in claim 2 , the decontamination system according to claim 1, characterized in that:
the decontamination agent is decontamination mist in an aerosol state, and
the decontamination agent transfer device discharges the decontamination mist discharged to the inside of the work room from the decontamination agent discharging device to an inside of the air supply space.

The reference signs in the parentheses for each of the above-described means indicate a correspondence relationship with concrete means described in embodiments described below.

### ADVANTAGEOUS EFFECTS OF INVENTION

According to the above-described configuration, the decontamination system is a system for decontaminating an air supply space constructed between an air filter for converting air supplied to an inside of a work room which maintains a sterile environment into clean air, and a straightening plate for straightening the clean air and supplying the straightened clean air to the inside of the work room. The decontamination system includes a decontamination agent discharging device provided in a work room, and a decontamination agent transfer device provided in the air supply space. When decontaminating an inside of the work room, the decontamination agent discharging device is operated to discharge the decontamination agent to the inside of the work room, and the decontamination agent transfer device is operated to suction, discharge, or circulate the decontamination agent between the work room and the air supply space. Consequently, the air supply space can be decontaminated as well as the work room.

Consequently, it is possible to provide a decontamination system having a simple structure using a decontamination agent supplied to a room targeted to be decontaminated without additionally providing a decontamination agent discharging device or the like in a space (air supply space) between an air filter and a straightening plate, the decontamination system being capable of efficiently decontaminating the air supply space in a short period of time.

Moreover, according to the above-described configuration, the straightening plate includes one or more porous sheets for straightening the clean air. Moreover, the decontamination agent transfer device includes an air duct having one end portion as a suction port and the other end portion as a discharge port, and an air-blowing fan provided inside the air duct. This decontamination agent transfer device is disposed so that the suction port is in contact with the porous sheet at an inside side of the air supply space. Consequently, the above-described operational advantageous effects can more specifically and more effectively be demonstrated.

Moreover, according to the above-described configuration, the decontamination agent may be decontamination mist in an aerosol state. Moreover, the decontamination agent transfer device discharges the decontamination mist discharged to the inside of the work room from the decontamination agent discharging device to an inside of the air supply space. Consequently, the above-described operational advantageous effects can more specifically and more effectively be demonstrated.

### BRIEF DESCRIPTION OF DRAWINGS

FIG. 1 is a schematic cross-sectional diagram observed from a front of an aspect of decontaminating a chamber of an isolator device with hydrogen peroxide mist in accordance with a conventional decontamination method;
FIG. 2 is a schematic cross-sectional diagram observed from a front of an aspect of decontaminating a chamber of an isolator device with hydrogen peroxide mist in accordance with a decontamination method using a decontamination system according to the present invention;
FIG. 3 is a cross-sectional diagram an aspect of decontaminating a space (air supply space) between an air supply filter unit and a straightening plate, observed from above an inside of the air supply space;
FIG. 4(A) is a front view diagram observed from a discharge port and FIG. 4(B) is fragmentary sectional view observed from a right-side surface, each which illustrates a structure of a decontamination agent transfer device used for the decontamination system according to the present invention;
FIG. 5 is an enlarged sectional view observed from a front of an aspect of decontaminating a space (air supply space) between an air supply filter unit and a straightening plate in FIG. 2; and
FIG. 6 is an enlarged sectional view observed from a front of an aspect of aerating the space (air supply space) between the air supply filter unit and the straightening plate after performing the decontamination illustrated in FIG. 5.

### DESCRIPTION OF EMBODIMENTS

First, prior to describing a decontamination method using an isolator device according to the present invention, a conventional decontamination method using a hydrogen peroxide mist will now be described. In the conventional decontamination method, and the decontamination method using the decontamination system according to the present invention both illustrated below, a hydrogen peroxide solution is used as a decontamination agent. Although a concentration of the hydrogen peroxide solution to be used is not limited in particular, in general, it is preferable to use the hydrogen peroxide solution of 30% to 35% by weight in consideration of handling of a hazardous material or the like. Moreover, the decontamination agent used in the the conventional decontamination method, and the decontamination method using the decontamination system according to the present invention is not limited to the hydrogen peroxide solution, and may be any liquid decontamination agent, such as an aqueous solution of peracetic acid.

In the present invention, the term "mist" is broadly interpreted as a state of a liquid droplet of a decontamination agent refined and floating in the air, a state of a gas and a liquid droplet of a decontamination agent in mixture, a state of the decontamination agent to repeat the change in phase between condensation and evaporation of a gas and a droplet, and the like. In terms of particle size as well, the mist is also broadly interpreted to include mists, fogs, and liquid droplets, which can be subclassified.

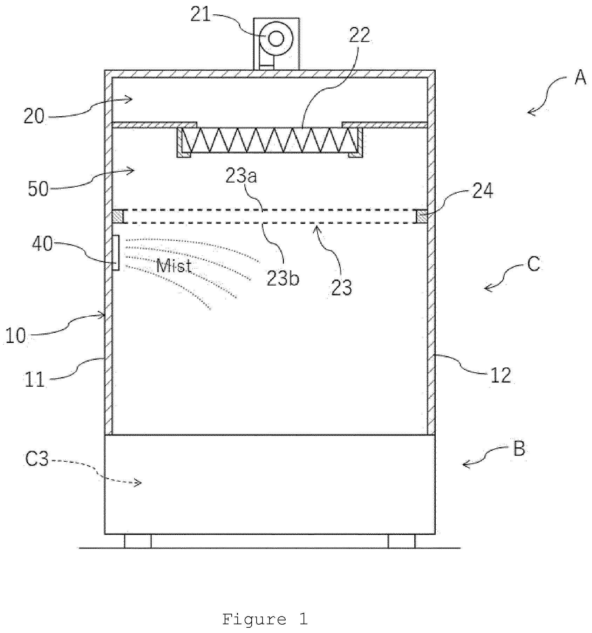

FIG. 1 is a schematic cross-sectional diagram observed from a front of an aspect of decontaminating a chamber of an isolator device with hydrogen peroxide mist in accordance with a conventional decontamination method. In FIG. 1, an isolator device A is constructed with a mount B placed on a floor surface, and an isolator main-body C mounted on this mount B. A periphery of the mount B is covered with a wall material made of a stainless steel metal plate, and an electrical and mechanical rooms (not illustrated) is housed in an inside thereof. The isolator main-body C includes a chamber 10, an air supply mechanism 20, and an exhaust mechanism 30. The chamber 10 is formed with a box constructed with a stainless steel metal plate and is airtightly shielded from an external environment in which an operator works except for restricted air intake and exhaust ports.

The air supply mechanism 20 includes an air supply blower 21 for supplying outside air into the chamber 10, an air supply filter unit 22 for filtering the air supplied from this air supply blower 21 and converting the air into clean air, and a straightening plate 23 disposed at a ceiling portion in a chamber 10 for straightening the clean air filtered by the air supply filter unit 22 and supplying the clean air into the chamber 10. In the present invention, a space between the air supply filter unit 22 and the straightening plate 23 illustrated in FIG. 1 is defined as an air supply space 50.

Inside the mount B, the exhaust mechanism 30 includes an exhaust port for exhausting the air in the chamber 10, an exhaust filter unit for filtering air to be exhausted, and an air exhaust blower for exhausting the air filtered by the exhaust filter unit (all not illustrated).

Herein, the straightening plate 23 will be described. The straightening plate 23 is constructed by tensely attaching two screen meshes 23a, 23b to a frame body 24. It is to be noted that the number screen meshes constructing the straightening plate 23 may not be particularly limited, and may be one or more. Moreover, the screen meshes 23a, 23b are generally woven fabrics made of synthetic filament yarns, and countless fine pores that communicates between front and back surfaces are formed by gaps between warp and weft yarns of this woven fabric. Consequently, a flow of air passing through the straightening plate 23 is straightened by these countless fine pores, thereby forming a unidirectional steady flow of clean air traveling from an upper side to a lower side inside the chamber.

The synthetic filament yarns forming these screen meshes 23a, 23b preferably have a filament diameter of 30 to 200 µm and preferably have an opening of 30 to 200 µm. Moreover, the screen meshes 23a, 23b may be made of any material, and polyethylene gauze is generally used in many cases. Thus, in the straightening plate 23, the openings of the screen meshes can be made as fine as approximately 30 to 200 µm. Consequently, the Reynolds number of the airflow passing through the screen meshes is approximately 2 to 10, and a unidirectional steady flow that is substantially a laminar flow can be formed.

Moreover, in FIG. 1, a mist discharging device 40 is provided on an upper portion of an internal wall surface of a left wall portion 11 in the chamber 10 as a decontamination agent discharging device. This mist discharging device 40 converts into hydrogen peroxide mist a hydrogen peroxide solution supplied through a supply pipe from a hydrogen peroxide tank installed outside the isolator device A (all not illustrated) and discharges the mist to inside the chamber 10. Consequently, the inside of the chamber 10 is decontaminated by the hydrogen peroxide mist as a decontamination agent.

It is to be noted that a mist generating mechanism of the mist discharging device 40 is not particularly limited. For example, the mist generating mechanism may be a single-fluid spray nozzle, a piezo high-pressure jet device, an immersion type ultrasonic atomizer, a disk-type atomizer, a disk-mesh type atomizer, or the like for directly forming the solution into mist. Alternatively, it may be an ejector, two-fluid spray nozzle, or the like for forming the solution into mist by using a high-pressure air or the like. On the other hand, output and mist discharging capacity, etc. of the mist discharging device 40 may be appropriately set, including the number thereof, in dependance with a volume, a shape, and the like of a room targeted to be decontaminated, such as a chamber.

Furthermore, a mist dispersion/diffusion device may be additionally provided in addition to the mist discharging device 40. The mist dispersion/diffusion device is configured with an ultrasonic vibration plate and to disperse and diffuse the hydrogen peroxide mist, discharged by the mist discharging device 40, by acting acoustic radiation pressure due to an ultrasonic vibration on the hydrogen peroxide mist.

In these configurations, the decontamination effect inside the chamber 10 can be effectively obtained in accordance with the decontamination operation illustrated in FIG. 1. However, the hydrogen peroxide mist cannot sufficiently be dispersed/diffused in the air supply space 50 between the air supply filter unit 22 and the straightening plate 23 due to the fine openings in the screen meshes 23a, 23b of the straightening plate 23. Accordingly, there has been a problem that the decontamination effect in this portion is weakened.

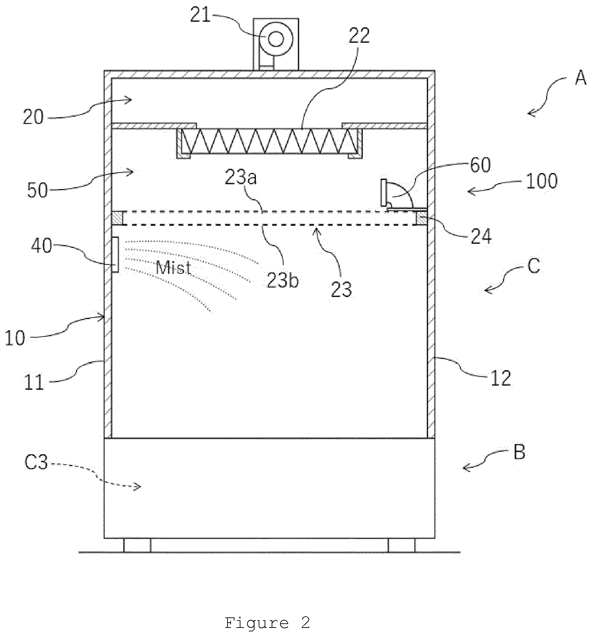

Therefore, the decontamination system according to the present invention will now be described based on the embodiments. FIG. 2 is a schematic cross-sectional diagram observed from a front of an aspect of decontaminating a chamber of an isolator device with hydrogen peroxide mist in accordance with a decontamination method using a decontamination system according to the present invention. In FIG. 2, contents of the isolator device A and the like are the same as those in FIG. 1, and the same reference signs are used therefor.

In FIG. 2, a decontamination system 100 according to the present invention is provided. The decontamination system 100 includes a mist discharging device 40 as a decontamination agent discharging device provided inside the chamber 10, and a decontamination agent transfer device 60 provided in the air supply space 50. The decontamination agent discharging device 40 is provided on the upper portion of the internal wall surface at the left wall portion 11 in the chamber 10, and has the same configuration as that illustrated in FIG. 1.

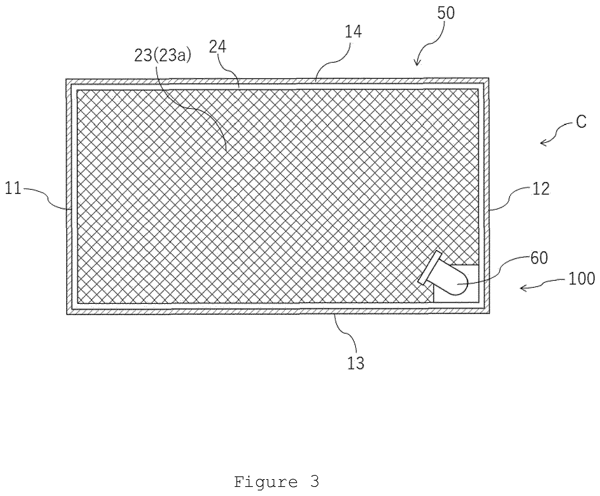

The decontamination agent transfer device 60 is provided in a state of being placed on the straightening plate 23. FIG. 3 is a cross-sectional diagram an aspect of decontaminating a space (air supply space) between an air supply filter unit and a straightening plate, observed from above inside of the air supply space. In FIG. 3, the decontamination agent transfer device 60 is provided, at a corner portion where a right wall portion 12 and a front wall portion 13 intersect, on an upper portion of the straightening plate 23, the straightening plate 23 provided at a ceiling portion of the chamber 10 so as to in contact with at an internal wall surface of four surfaces, i.e., a left wall portion 11, the right wall portion 12, the front wall portion 13, and a back wall portion 14.

It is to be noted that a position of the decontamination agent transfer device 60 in the air supply space 50 is not particularly limited, and it may be disposed in consideration of: a relationship with a position of the mist discharging device 40 provided inside the chamber 10; a position of an important sterilization process, work (pass line), and the like in the isolator device; and a state of dispersion and diffusion of a hydrogen peroxide mist inside the air supply space 50. Moreover, the number of decontamination agent transfer devices 60 is not be limited to one, and may be appropriately set depending on a relationship with a volume of the air supply space 50, and the like.

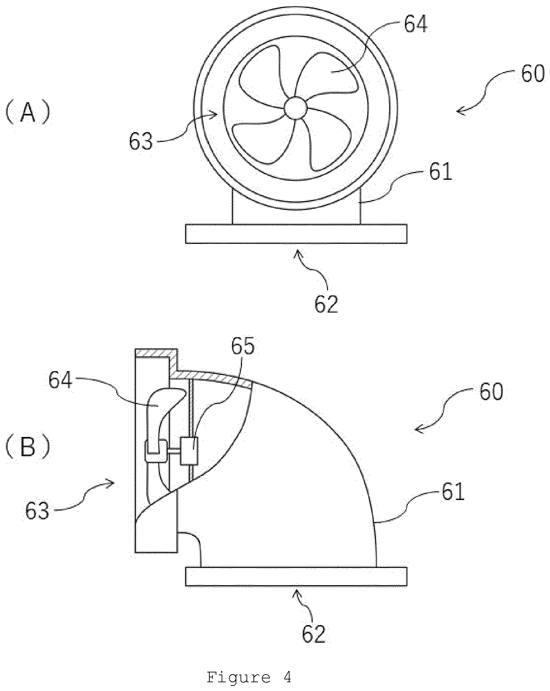

Herein, a structure of the decontamination agent transfer device 60 will now be described. FIG. 4(A) is a front view diagram observed from a discharge port and FIG. 4(B) is fragmentary sectional view observed from a right-side surface, each which illustrates a structure of a decontamination agent transfer device used for the decontamination system according to the present invention. In FIG. 4, the decontamination agent transfer device 60 includes an air duct 61 formed by bending a circular pipe at substantially 90° as a main body, one end portion opened downward as a suction port 62, and the other end portion opened in a horizontal direction as a discharge port 63. Moreover, near the discharge port 63, an air-blowing fan 64 and a drive motor 65 are disposed to be inserted. It is to be noted that a check valve and the like are not required. Moreover, in FIG. 4, a power supply and wiring are omitted.

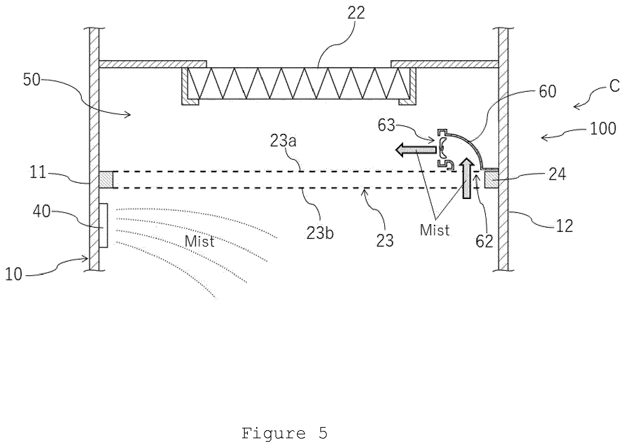

Next, an action of the decontamination agent transfer device 60 in decontamination and aeration operations will be described. First, an operation for decontaminating the inside of the chamber 10 and the inside of the air supply space 50 will be described. FIG. 5 is an enlarged sectional view observed from a front of an aspect of decontaminating a space (air supply space) between an air supply filter unit and a straightening plate in FIG. 2.

In FIG. 5, the decontamination agent transfer device 60 is disposed at the right wall portion 12 side, inside of the air supply space 50 so that the suction port 62 is in contact with the screen mesh 23a on the upper portion of the straightening plate 23. In this state, inside the chamber 10, a hydrogen peroxide mist is discharged from the mist discharging device 40. Accordingly, as a state inside a chamber 10, the hydrogen peroxide mist is dispersed and diffused in every corner. Moreover, in FIG. 5, the air-blowing fan 64 in the decontamination agent transfer device 60 is operating. Accordingly, the hydrogen peroxide mist inside the chamber 10 is in a state of being sucked from the suction port 62 and dispersed and diffused from the discharge port 63 into the air supply space 50.

In addition, since the suction port 62 of the decontamination agent transfer device 60 is in contact with the screen mesh 23a, the hydrogen peroxide mist inside the chamber 10 is sucked through the two screen meshes 23a, 23b of the straightening plate 23. Accordingly, it is preferable to adjust the output of the air-blowing fan 64 with respect to the number of screen meshes and the openings in accordance with the capacity of the air supply space 50. By maintaining such a state, the inside of the chamber 10 and the inside of the air supply space 50 can be efficiently decontaminated in a short period of time.

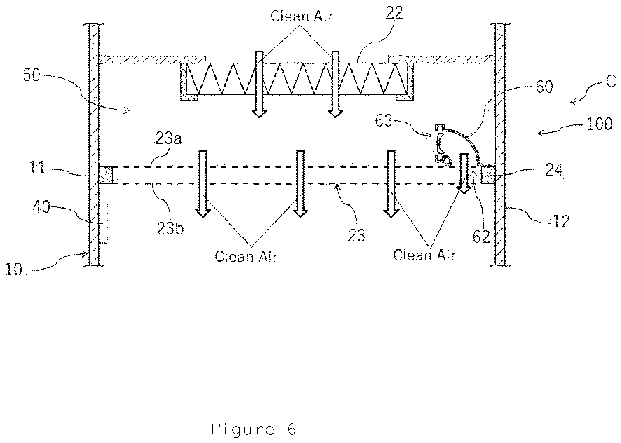

Next, an operation of performing aeration after decontaminating the inside of the chamber 10 and the inside of the air supply space 50 will be described. FIG. 6 is an enlarged sectional view observed from a front of an aspect of aerating the space (air supply space) between the air supply filter unit and the straightening plate after performing the decontamination illustrated in FIG. 5.

In FIG. 6, the operation of decontamination has been completed and then an operation of aeration is being performed. Inside the chamber 10, discharge of the hydrogen peroxide mist from the mist discharging device 40 has stopped. The operation of an air-blowing fan 64 in the decontamination agent transfer device 60 has also been stopped. Moreover, the air supply blower 21 and the air exhaust blower (both not illustrated) are being operated.

In this state, clean air is supplied from the air supply blower 21 to the inside of the air supply space 50 through the air supply filter unit 22, and aeration is performed. Moreover, inside the air supply space 50, the clean air is flowing into the inside of the chamber 10 through the two screen meshes 23a, 23b of the straightening plate 23 due to pressurization acted by the air supply blower 21 and the suction acted by the air exhaust blower that are operating simultaneously. It is to be noted that the clean air flows into portions of the aeration and the screen meshes 23a, 23b with which the suction port 62 of the decontamination agent transfer device 60 is in contact, the aeration is also performed as in other portions.

The clean air flowing into the inside of the chamber 10 aerates the inside of the chamber 10. The air inside the chamber 10 is introduced into an external hydrogen peroxide decomposition catalyst device (not illustrated) from the air exhaust blower through the exhaust filter unit. By maintaining such a state, the inside of the chamber 10 and the inside of the air supply space 50 can be efficiently aerated in a short period of time.

As described above, according to the present invention, it is possible to provide the decontamination system having a simple structure using a decontamination agent supplied to a room targeted to be decontaminated without additionally providing a decontamination agent discharging device or the like in a space (air supply space) between an air filter and a straightening plate, the decontamination system being capable of efficiently decontaminating the air supply space in a short period of time.

### REFERENCE SIGNS LIST

- A: Isolator device,
- B: Mount,
- C: Isolator main-body,
- 10: Chamber,
- 11 to 14: Chamber wall portion,
- 20: Air supply mechanism,
- 30: Exhaust mechanism,
- 24: Frame body,
- 21: Air supply blower,
- 22: Air supply filter unit,
- 23: Straightening plate,
- 23a, 23b: Screen mesh,
- 40: Mist discharging device,
- 50: Air supply space,
- 60: Decontamination agent transfer device,
- 61: Air duct,
- 62: Suction port,
- 63: Discharge port,
- 64: Air-blowing fan,
- 65: Drive motor, and
- 100: Decontamination system.

## Claims

1. An isolator device (A) comprising a decontamination system (100) for decontaminating an air supply space (50) constructed between an air filter (22) for converting air supplied to an inside of a work room (10) which is configured to maintain a sterile environment into clean air, and a straightening plate (23) for straightening the clean air and supplying the straightened clean air to the inside of the work room (10),
further comprising said work room (10), said air supply space (50), said air filter (22), and said straightening plate (23);
the decontamination system comprising a decontamination agent discharging device (40) provided in the work room (10), and a decontamination agent transfer device (60) provided in the air supply space (50), **characterized in that**
the decontamination system is configured so that when decontaminating the inside of the work room (10), the decontamination agent discharging device (40) is operated to discharge a decontamination agent to the inside of the work room (10), and the decontamination agent transfer device (60) is operated to suction, discharge, or circulate the decontamination agent between the work room (10) and the air supply space (50),
and thereby the air supply space (50) is decontaminated as well as the work room (10);
the straightening plate (23) comprises one or more porous sheets for straightening the clean air, and
**characterized in that**
the decontamination agent transfer device (60) comprises an air duct (61) having one end portion as a suction port (62) and the other end portion as a discharge port (63), and an air-blowing fan (64) provided inside the air duct (61), and is disposed so that the suction port (62) is in contact with the porous sheet at an inside side of the air supply space (50) .

2. The isolator device (A) according to claim 1 **characterized in that**
the decontamination agent is decontamination mist in an aerosol state, and
the decontamination agent transfer device (60) discharges the decontamination mist discharged to the inside of the work room (10) from the decontamination agent discharging device (40) to an inside of the air supply space (50).

## Patentansprüche

1. Isolatorvorrichtung (A), umfassend ein Dekontaminationssystem (100) zur Dekontamination eines Luftzufuhrraums (50), der zwischen einem Luftfilter (22) - der dazu dient, die einem Innenraum eines Arbeitsraums (10), der zur Aufrechterhaltung einer sterilen Umgebung konfiguriert ist, zugeführte Luft in saubere Luft umzuwandeln - und einer Ausrichtplatte (23) - die dazu dient, die saubere Luft auszurichten und die ausgerichtete saubere Luft dem Innenraum des Arbeitsraums (10) zuzuführen - ausgebildet ist,
ferner umfassend den Arbeitsraum (10), den Luftzufuhrraum (50), den Luftfilter (22) und die Ausrichtplatte (23);
wobei das Dekontaminationssystem eine im Arbeitsraum (10) vorgesehene Dekontaminationsmittel-Ausgabevorrichtung (40) und eine im Luftzufuhrraum (50) vorgesehene Dekontaminationsmittel-Übertragungsvorrichtung (60) umfasst, **dadurch gekennzeichnet, dass**
das Dekontaminationssystem so konfiguriert ist, dass bei der Dekontamination des Innenraums des Arbeitsraums (10) die Dekontaminationsmittel-Ausgabevorrichtung (40) so betrieben wird, dass sie ein Dekontaminationsmittel in das Innere des Arbeitsraums (10) abgibt, und die Dekontaminationsmittel-Übertragungsvorrichtung (60) so betrieben wird, dass sie das Dekontaminationsmittel zwischen dem Arbeitsraum (10) und dem Luftzufuhrraum (50) ansaugt, abgibt oder umwälzt,
wodurch sowohl der Luftzufuhrraum (50) als auch der Arbeitsraum (10) dekontaminiert werden;
die Ausrichtplatte (23) eine oder mehrere poröse Platten zum Ausrichten der sauberen Luft umfasst, und
**dadurch gekennzeichnet, dass**
die Dekontaminationsmittel-Übertragungsvorrichtung (60) einen Luftkanal (61) umfasst, dessen einer Endabschnitt als Ansaugöffnung (62) und dessen anderer Endabschnitt als Auslassöffnung (63) ausgebildet ist, sowie ein im Inneren des Luftkanals (61) vorgesehenes Gebläse (64), und so angeordnet ist, dass die Ansaugöffnung (62) an einer Innenseite des Luftzufuhrraums (50) mit der porösen Platte in Kontakt steht.

2. Isolatorvorrichtung (A) nach Anspruch 1, **dadurch gekennzeichnet, dass**
das Dekontaminationsmittel ein Dekontaminationsnebel in Aerosolform ist und
die Dekontaminationsmittel-Übertragungsvorrichtung (60) den von der Dekontaminationsmittel-Ausgabevorrichtung (40) in das Innere des Arbeitsraums (10) abgegebenen Dekontaminationsnebel in das Innere des Luftzufuhrraums (50) leitet.

## Revendications

1. Dispositif d'isolation (A) comprenant un système de décontamination (100) destiné à décontaminer un espace d'alimentation en air (50) formé entre un filtre à air (22) destiné à transformer en air pur l'air fourni à l'intérieur d'une salle de travail (10) configurée pour maintenir un environnement stérile, et une plaque de redressement (23) destinée à redresser l'air pur et à fournir l'air pur redressé à l'intérieur de la salle de travail (10), comprenant en outre ladite salle de travail (10), ledit espace d'alimentation en air (50), ledit filtre à air (22) et ladite plaque de redressement (23) ;
le système de décontamination comprenant un dispositif d'évacuation d'agent de décontamination (40) prévu dans la salle de travail (10), et un dispositif de transfert d'agent de décontamination (60) prévu dans l'espace d'alimentation en air (50), **caractérisé en ce que**
le système de décontamination est configuré de telle sorte que, lors de la décontamination de l'intérieur de la salle de travail (10), le dispositif d'évacuation d'agent de décontamination (40) est actionné pour évacuer un agent de décontamination à l'intérieur de la salle de travail (10), et le dispositif de transfert d'agent de décontamination (60) est actionné pour aspirer, évacuer ou faire circuler l'agent de décontamination entre la salle de travail (10) et l'espace d'alimentation en air (50),
ce qui permet de décontaminer à la fois l'espace d'alimentation en air (50) et la salle de travail (10) ;
la plaque de redressement (23) comprend une ou plusieurs feuilles poreuses destinées à redresser l'air pur, et
**caractérisé en ce que**
le dispositif de transfert d'agent de décontamination (60) comprend un conduit d'air (61) dont une extrémité forme un orifice d'aspiration (62) et l'autre extrémité un orifice d'évacuation (63), ainsi qu'un ventilateur de soufflage d'air (64) disposé à l'intérieur du conduit d'air (61), et est agencé de telle sorte que l'orifice d'aspiration (62) soit en contact avec la feuille poreuse située sur un côté intérieur de l'espace d'alimentation en air (50).

2. Dispositif d'isolation (A) selon la revendication 1, **caractérisé en ce que**
l'agent de décontamination est un brouillard de décontamination sous forme d'aérosol, et
le dispositif de transfert d'agent de décontamination (60) évacue le brouillard de décontamination évacué à l'intérieur de la salle de travail (10) par le dispositif d'évacuation d'agent de décontamination (40), vers un intérieur de l'espace d'alimentation en air (50).
